Europäisches Patentamt

European Patent Office

Office européen des brevets

(11) Publication number: **0 087 980**
**B1**

(12) **EUROPEAN PATENT SPECIFICATION**

(45) Date of publication of patent specification: **28.05.86**

(51) Int. Cl.⁴: **C 07 C 5/03, C 07 C 5/08**

(21) Application number: **83301111.7**

(22) Date of filing: **02.03.83**

(54) **Method of selective hydrogenation of hydrocarbons.**

(30) Priority: **02.03.82 JP 33652/82**

(43) Date of publication of application:
**07.09.83 Bulletin 83/36**

(45) Publication of the grant of the patent:
**28.05.86 Bulletin 86/22**

(84) Designated Contracting States:
**DE FR GB IT NL**

(56) References cited:
**GB-A-1 011 270**
**GB-A-1 161 645**
**GB-A-1 296 943**

(73) Proprietor: **SUMITOMO CHEMICAL COMPANY,
LIMITED**
**15 Kitahama 5-chome Higashi-ku**
**Osaka-shi Osaka 541 (JP)**

(72) Inventor: **Araki, Masashi**
**No. 1353-4 Shiizu Ichihara-shi**
**Chiba (JP)**
Inventor: **Higashio, Yasuhiko**
**No. 11-11 Aobadai 3-chome Ichihara-shi**
**Chiba (JP)**

(74) Representative: **Diamond, Bryan Clive et al**
**Gee & Co. Chancery House Chancery Lane**
**London WC2A 1QU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention). ·

Courier Press, Leamington Spa, England.

# 0 087 980

## Description

This invention relates to a method of selectively hydrogenating dienes and acetylenes into monoenes. More particularly, the invention relates to a method of catalytically converting only unsaturated hydrocarbons having two or more double bonds and/or one or more triple bonds in each molecule (hereinafter referred to as "highly unsaturated hydrocarbons") into corresponding hydrocarbons of low unsaturation degree without also causing isomerization of the double bonds.

A method is known wherein a mixture of unsaturated hydrocarbons having 4 or more carbon atoms together with highly unsaturated hydrocarbons is reacted with hydrogen in the presence of a hydrogenation catalyst to selectively hydrogenate the highly unsaturated hydrocarbons into corresponding hydrocarbons of low unsaturation degree. Thus, it is possible on an industrial scale to selectively hydrogenate dienes and acetylenes only by reacting a mixture of unsaturated hydrocarbons having 4 carbon atoms containing dienes such as butadiene or methylallenes, acetylenes such as dimethylacetylene, ethylacetylene or vinylacetylene and monoolefins such as 1-butene, 2-butene or isobutene with hydrogen in the presence of a hydrogenation catalyst such as palladium, platinum or nickel.

However, in such a known selective hydrogenation method, there is a problem in that intramolecular transfer of double bonds easily occurs simultaneously with the selective hydrogenation of highly unsaturated bonds (such as two or more double bonds and one or more triple bonds) whereby the composition of the reaction product is greatly changed. For example, during selective hydrogenation of unsaturated hydrocarbons having 4 carbon atoms containing, for example, butadienes and butynes, isomerization of 1-butene into 2-butene easily occurs simultaneously with the selective hydrogenation of butadienes and butynes into butenes, whereby the concentration of 1-butene in the reaction product is greatly reduced.

1-Butene is important as a monomer for producing polyolefins. Hence, from the viewpoint of effective utilization of 1-butene in a mixture of hydrocarbons having 4 carbon atoms, it is desirable to devise a method capable of selectively hydrogenating highly unsaturated hydrocarbons only without isomerization.

The invention provides a method of selectively hydrogenating at least one unsaturated hydrocarbon compound having 2 or more double bonds or 1 or more triple bond in the molecule, which is admixed with at least one hydrocarbon compound having 4 or more carbon atoms, by contact with gaseous hydrogen in the vapor phase in the presence of a catalyst in a fixed bed reaction vessel, characterised in that the hydrogen is supplied, in an amount of 1 to 3 moles per mole of said unsaturated hydrocarbon at at least two points along the length of the reaction vessel, which may consist of two or more reaction vessels connected in series, provided that the proportion of the hydrogen supplied at the second or subsequent point is at least 5% by weight of the amount supplied at the previous point.

When performing the hydrogenation of hydrocarbons using a fixed bed reaction vessel, the whole necessary amount of hydrogen gas is usually supplied to the inlet of the reaction vessel together with the starting hydrocarbons. However, in selectively hydrogenating highly unsaturated hydrocarbons by bringing a mixture of hydrocarbons of low unsaturation degree having 4 or more carbon atoms containing the highly unsaturated hydrocarbons into contact with hydrogen in the presence of a catalyst using a fixed bed reaction, when the hydrogenation reaction is performed, as is customary, by supplying hydrogen gas to only the inlet of the reaction vessel, intramolecular transfer of double bonds easily occurs simultaneously with the desired hydrogenation reaction; we have surprisingly found that by supplying the hydrogen gas in the manner specified for the invention, only the highly unsaturated hydrocarbons are selectively hydrogenated without the intramolecular transfer of double bonds.

As a method of selectively hydrogenating highly unsaturated hydrocarbons without isomerization of olefins, it is known to perform the selective hydrogenation reaction using $H_2$ gas containing a large amount of carbon monoxide gas (Japanese Patent Publication No. 39808/71); to hydrogenate in a vapor-liquid mixed phase in a first step and then hydrogenate in a liquid phase in a second step (Japanese Patent Publication No. 16082/77); and to use a specific catalyst (Japanese Patent Publication No. 28922/75 and German Patent No. 2,108,276). However, these known methods have the respective faults that it is necessary to use expensive carbon monoxide to use a specific catalyst, and that the process becomes complicated.

On the other hand, this invention provides a method of selectively hydrogenating highly unsaturated hydrocarbons only without appreciable isomerization of olefins and in a relatively simple manner. Hence, the invention is industrially useful.

British Patent Specification No. 1,161,645 describes a method for selectively hydrogenating acetylenes (having triple bonds) in a mixture of hydrocarbons having 4 or 5 carbon atoms, by contact with gaseous hydrogen in the presence of a solid catalyst, wherein when the mixture contains large quantities of acetylenes (amounts of up to about 0.5% by weight are shown in the Examples) then some of the hydrogen is added before or at the beginning of the reaction and the remainder at 2 or more points during·the reaction. The main hydrocarbon content is illustrated as butadienes (having two double bonds) which are *not* hydrogenated.

British Patent Specification No. 1,296,943 describes a method for selectively hydrogenating unsaturated hydrocarbons, including alkadienes and acetylenes. In mixtures of C3—C10 hydrocarbons, by contact with gaseous hydrogen in a fixed catalyst bed; the reaction is carried out in two stages, and at the

2

outlet from the first stage reaction zone the vaporized fraction is condensed and the liquid fraction is again treated in a fixed catalyst bed with hydrogen gas but it is not clear whether fresh hydrogen needs to be added at that stage.

Both the aforesaid processes are carried out with the hydrocarbons in the liquid phase. In the present invention the hydrocarbon mixture is in the gaseous (vapor) phase; and it is carried out in a single reaction step without any intermediate separation step and whereby alkadienes are hydrogenated as well as acetylenes. We have found that the supply of the hydrogen gas at several points is especially effective in a vapor phase reaction.

As regards the manner of supply of the hydrogen gas at multiple locations, in the invention, the gas may be supplied through multiple gas inlets disposed along the flow direction of a fixed bed reaction vessel packed with a catalyst, or, more preferably, a catalyst layer packed into a fixed bed reaction vessel is split into several layers along the flow direction of the reaction vessel and the hydrogen gas is separately supplied to several zones which contain no catalyst between the respective catalyst layers.

Also, two or more fixed bed reaction vessels can be connected in series and hydrogen gas separately supplied to each reaction vessel; and a heat exchanger, or other control means, may be disposed between the reaction vessels, whereby the reaction temperature and/or reaction pressure of each reaction vessel thus connected in series can be independently controlled at preferred conditions for performing the reaction.

The hydrogen gas is supplied at several points along the flow direction of the fixed bed reaction vessel. As the number of points of supply of the hydrogen gas is increased, the result of the reaction is improved but the procedure becomes more complicated and troublesome. Therefore, it is preferred to supply the hydrogen gas at only two or three points.

The proportion of each hydrogen gas supplied varies with the position of the inlet thereof but it is necessary that the amount of hydrogen gas at the second or later point(s) be at least 5% and preferably 5 to 100% or the weight of hydrogen gas in the previous split; thus the selective hydrogenation of highly unsaturated hydrocarbons can be performed, without appreciable isomerization of olefins.

There is no particular restriction on the positions of supply of the hydrogen gas, but usually they are disposed so that a part of the hydrogen gas is split off before the hydrocarbons are brought into contact with a catalyst and that the second and later inlet portions are disposed along the flow direction of the fixed bed reaction vessel at almost equal intervals. Also, in the case of performing the reaction using a fixed bed reaction vessel having several catalyst layers disposed along the flow direction or several fixed bed reaction vessels connected in series along the flow direction, the size of the respective catalyst-packed layers or the size of the respective individual reaction vessels is usually (but not essentially) selected to be substantially the same, and the hydrogen gas supply is split before each catalyst layer or each fixed bed reaction vessel.

In this invention, any types of isothermal-type reaction vessels or heat insulating-type reaction vessels can be used as the fixed bed reaction vessel, or a combination of both types can also be employed.

As the catalyst used in this invention, a nickel catalyst, a palladium catalyst or a platinum catulyst are useful, with the palladium catalyst being preferred. The palladium catalyst may be used in the form of palladium black, palladium-on-carbon, or palladium-on-alumina. Usually, a catalyst having 0.02 to 2% by weight of palladium supported on a carrier such as alumina is used.

Examples of the mixture of hydrocarbons of low unsaturation degree having 4 or more carbon atoms containing highly unsaturated hydrocarbons are a mixture of $C_4$ hydrocarbons composed of so-called $C_4$ fractions such as butadiene, butane or butene, obtained by steam cracking of naphtha; a mixture of $C_4$ hydrocarbons called spent BB fractions, i.e., the foregoing $C_4$ fractions from which a major part of butadiene has been removed by extraction; a mixture of $C_4$ hydrocarbons mainly composed of 1-butene and 2-butene obtained by further removing isobutylene from the spent BB fractions; and a mixture of hydrocarbons mainly composed of $C_5$ hydrocarbons such as isoprene.

As highly unsaturated hydrocarbons present in these hydrocarbon mixtures, there are propadiene, methylacetylene, 1,2-butadiene, 1,3-butadiene, ethylacetylene, vinylacetylene and 1,3-pentadiene for example.

The reaction in the method of this invention is in the vapor phase.

In the method of the invention, a gaseous hydrocarbon mixture emerging from the top of a distilling column may be directly supplied to the hydrogenation reaction vessel. Also, a gaseous mixture after the hydrogenation reaction may be supplied to a distilling column in a gaseous state without being liquified to perform the separation of hydrocarbons. By this manner, the process can be simplified and energy required can be saved.

In the hydrogenation reaction employed in the method of this invention, the reaction temperature is usually −20°C to 150°C and the reaction pressure is usually atmospheric pressure to 30 bars. It is, however, preferred that the reaction temperature be 20 to 100°C and the reaction pressure be 2 to 15 bars. In the vapor phase the maximum pressure corresponding to 150°C is 30 bars and that corresponding to 100°C is 15 bars. The total amount of hydrogen gas supplied is in the range of 1 to 3 moles, preferably 1.1 to 2.0 moles per mole of the highly unsaturated hydrocarbons in the starting hydrocarbon mixture.

The method of this invention will be further explained by the following examples.

3

**0 087 980**

Example 1

A $C_4$ hydrocarbon mixture having the composition as shown in Table 1 was used as the starting material. Also, a catalyst having 0.3% by weight of palladium supported on an alumina carrier was used. The palladium-on-alumina catalyst was packed into each of two reaction tubes of 20 mm inside diameter and length 50 cm, in an amount of 75 ml. The reaction tubes were connected with each other in series and placed vertically. Under the conditions of a reaction temperature of 50°C and a reaction pressure of 4 bars, the foregoing $C_4$ hydrocarbon mixture was introduced into the first reaction tube at a rate of 1.18 kg/hr. and also hydrogen gas was introduced into the inlet of the first reaction tube at a rate of 7.5 liters (calculated as NTP)/hr. and into the inlet of the second reaction tube at a rate of 1.8 liters (calculated as NTP)/hr. to perform the vapor-phase hydrogenation. The composition of the reaction product thus obtained is shown in Table 1.

TABLE 1

| Compound | Composition of starting material (mole%) | Composition of product (mole%) |
|---|---|---|
| Propane, Propadiene | 0.02 | 0.29 |
| i-Butane | 4.73 | 4.74 |
| n-Butane | 11.89 | 11.89 |
| i-Butene | 46.83 | 46.84 |
| 1-Butene | 24.21 | 24.36 |
| 2-Butene | 11.16 | 11.87 |
| 1,3-Butadiene | 0.76 | 0 |
| Propadiene | 0.26 | 0 |
| 1,2-Butadiene | 0.05 | 0 |
| Vinylacetylene | 0.08 | 0 |

By supplying the hydrogen gas at two points, butadiene as well as other dienes and acetylenes could be completely hydrogenated without loss of 1-butene, as shown in Table 1.

Examples 2 to 4 and comparative example 1

The same catalyst and reaction apparatus was used as in Example 1, and the hydrogenation was performed under the same reaction conditions as in Example 1 except that the hydrogen gas was supplied at the rates as shown in Table 2. The results obtained are shown in Table 2.

TABLE 2

| | (A)* | (B)* | (C)* | (D)* |
|---|---|---|---|---|
| Example 2 | 8.5 | 1.0 | 12 | 0.1 |
| Example 3 | 5.5 | 4.9 | 9 | 0.8 |
| Example 4 | 3.5 | 7.7 | 21 | 3.2 |
| Comparative Example 1 | 13.8 | 0 | 16 | 14.5 |

(A)*: Hydrogen supply rate at the inlet of the first reaction tube in liters (cald. as NTP)/hr.
(B)*: Hydrogen supply rate at the inlet of the 2nd reaction tube in liters (cald. as NTP)/hr.
(C)*: Residual amount of butadiene (mole ppm).
(D)*: Conversion ratio of 1-butene (%).

**Claims**

1. A method of selectively hydrogenating at least one unsaturated hydrocarbon compound having 2 or

4

more double bonds or 1 or more triple bond in the molecule, which is admixed with at least one hydrocarbon compound having 4 or more carbon atoms, by contact with gaseous hydrogen in the vapor phase in the presence of a catalyst in a fixed bed reaction vessel, characterised in that the hydrogen is supplied, in an amount of 1 to 3 moles per mole of said unsaturated hydrocarbon at at least two points along the length of the reaction vessel, which may consist of two or more reaction vessels connected in series, provided that the proportion of the hydrogen supplied at the second or subsequent point is at least 5% by weight of the amount supplied at the previous point.

2. A method as claimed in Claim 1, characterised in that the flow rate of hydrogen gas at the second or later supply point along the flow direction of said reaction vessel is 5 to 100% of that at the previous point.

3. A method as claimed in Claim 1 or 2, which is carried out in a fixed bed reaction vessel containing several catalyst layers split into several zones along the flow direction and the hydrogen gas is independently supplied to each zone.

4. A method as claimed in Claim 1, 2 or 3, wherein the total amount of hydrogen gas supplied is 1.1 to 2.0 moles per mole of said unsaturated hydrocarbons in the starting hydrocarbon mixture.

5. A method as claimed in any of Claims 1 to 4, wherein a palladium catalyst is used as the catalyst.

6. A method as claimed in any preceding claim, wherein propadiene, methylacetylene, 1,2- of 1,3-butadiene, ethylacetylene, vinylacetylene and/or 1,3-pentadiene is hydrogenated.

## Patentansprüche

1. Verfahren zur selektiven Hydrierung von mindestens einer ungesättigten Kohlenwasserstoffverbindung mit zwei oder mehr Doppelbindungen oder mit einer oder mehr Dreifachbindungen im Molekül unter Zumischung von mindestens einer Kohlenwasserstoffverbindung mit vier oder mehr Kohlenstoffatomen, durch Kontakt mit gasförmigen Wasserstoff in der Gasphase in Gegenwart eines Katalysators in einem Festbett-Reaktionsgefäß, dadurch gekennzeichnet, daß der Wasserstoff in einer Menge von 1 bis 3 Mol pro Mol des ungesättigten Kohlenwasserstoffs an mindestens zwei Stellen entlang dem Reaktionsgefäß zugeführt wird, wobei das Reaktionsgefäß aus zwei oder mehr in Reihe geschalteten Reaktionsgefäßen bestehen kann, wobei der Anteil des an der zweiten oder nachfolgenden Stelle zugeführten Wasserstoffs mindestens 5% der Menge ausmacht, die an der vorhergehenden Stelle zugeführt wird.

2. Verfahren nach Anspruch 1, dadurch gekennzeichnet, daß die Strömungsgeschwindigkeit des Wasserstoffgases an der zweiten oder einer späteren Zuführungsstelle entlang der Strömungsrichtung des Reaktionsgefäßes 5 bis 100% der Strömungsgeschwindigkeit an der vorherigen Stelle ausmacht.

3. Verfahren nach Anspruch 1 oder 2, das in einem Festbett-Reaktionsgefäß durchgeführt wird, welches mehrere Katalysatorschichten enthält, die in mehrere Zonen entlang der Strömungsrichtung aufgespalten sind, und wobei das Wasserstoffgas in jeder Zone unabhängig zugeführt wird.

4. Verfahren nach Anspruch 1, 2 oder 3, wobei die Gesamtmenge des zugeführten Wasserstoffgases 1,1 bis 2,0 Mol pro Mol der ungesättigten Kohlenwasserstoffe im Ausgangskohlenwasserstoffgemisch beträgt.

5. Verfahren nach jedem der Ansprüche 1 bis 4, wobei als Katalysator ein Palladium-Katalysator verwendet wird.

6. Verfahren nach jedem der vorangehenden Ansprüche, wobei Propadien, Methylacetylen, 1,2- oder 1,3-Butadien, Äthylacetylen, Vinylacetylen und/oder 1,3-Pentadien hydriert werden.

## Revendications

1. Procédé pour hydrogéner sélectivement au moins un hydrocarbure insaturé comportant 2 ou plus de deux doubles liaisons ou 1 ou plus d'une triple liaison dans la molécule, qui est en mélange avec au moins un hydrocarbure ayant 4 on plus de quatre atomes de carbone, par contact avec l'hydrogène gazeux en phase vapeur en présence d'un catalyseur dans un réacteur à lit fixe, procédé caractérisé en ce que l'hydrogène est introduit en une quantité de 1 à 3 mol par mol dudit hydrocarbure insaturé, en au moins deux points le long du réacteur, lequel peut consister en deux ou plusieurs réacteurs liés en série, à la condition que la proportion d'hydrogène introduite au second point ou en un point subséquent représente au moins 5% en poids de la quantité introduite au point précédent.

2. Procédé selon la revendication 1, caractérisé en ce que le débit d'introduction d'hydrogène gazeux au second point d'introduction ou en un point ultérieur dans la direction d'écoulement dans ledit réacteur représente 5 à 100% du débit d'écoulement au point précédent.

3. Procédé selon la revendication 1 ou 2, qui est mis en oeuvre dans un réacteur à lit fixe contenant plusieurs couches de catalyseur séparées en plusieurs zones dans la direction d'écoulement, l'hydrogène gazeux étant introduit indépendamment dans chaque zone.

4. Procédé selon la revendication 1, 2 ou 3, dans lequel la quantité totale d'hydrogène gazeux introduit est de 1,1 à 2,0 mol par mol desdits hydrocarbures insaturés présents dans le mélange des hydrocarbures de départ.

5. Procédé selon l'une quelconque des revendications 1 à 4, dans lequel on utilise comme catalyseur un catalyseur au palladium.

6. Procédé selon l'une quelconque des revendications précédentes, dans lequel on hydrogène du propadiène, du méthylacétylène, le 1,2-butadiène ou le 1,3-butadiène, l'éthylacétylène, le vinylacétylène et/ou le 1,3-pentadiène.